# EUROPEAN PATENT SPECIFICATION

(11) **EP 1 290 228 B1**
(45) Date of publication and mention of the grant of the patent: **09.11.2005**
(21) Application number: 01944762.2
(22) Date of filing: 14.06.2001
(51) Int. Cl.: C12Q 1/68

(54) **REVERSE DETECTION FOR IDENTIFICATION AND/OR QUANTIFICATION OF NUCLEOTIDE TARGET SEQUENCES ON BIOCHIPS**
UMGEKEHRTER NACHWEIS ZUR IDENTIFIZIERUNG UND/ODER QUANTIFIZIERUNG VON NUKLEOTID-ZIELSEQUENZEN MITTELS BIOCHIPS
DETECTION INVERSE POUR L'IDENTIFICATION ET/OU LA QUANTIFICATION DES SEQUENCES NUCLEOTIDIQUES CIBLES SUR DES BIOPUCES

(30) Priority: 14.06.2000 EP 00870127
(43) Date of publication of application: 12.03.2003
(73) Proprietor: Eppendorf Array Technologies SA (EAT), 5000 Namur (BE)
(72) Inventor: REMACLE, José, B-5020 Malonne (BE); ART, Muriel, B-5000 Namur (BE); LOCKMAN, Laurence, B-6600 Bastogne (BE); ZAMMATTEO, Nathalie, B-5024 Gelbressee (BE)
(74) Representative: Van Malderen, Joelle
(86) International application number: PCT/BE2001/000101
(87) International publication number: WO 2001/096592

(56) References cited:
- EP-A- 0 420 260
- WO-A-00/43538
- WO-A-00/53317
- WO-A-00/72018
- WO-A-93/03182
- WO-A-93/25563
- WO-A-96/07917
- WO-A-97/27317
- WO-A-97/31256
- WO-A-98/11253
- WO-A-99/28497
- ARMOUR ET AL: "MEASUREMENT OF LOCUS COPY NUMBER BY HYBRIDISATION WITH AMPLIFIABLE PROBES" NUCLEIC ACIDS RESEARCH,GB,OXFORD UNIVERSITY PRESS, SURREY, vol. 28, no. 2, 2000, pages 605-609, XP002138423 ISSN: 0305-1048

## Description

### Field of the invention

The present invention provides a method for the identification and/or the quantification of one or more target nucleotide sequences present in a sample and which allows their discrimination with homologous sequences, especially the identification and quantification of specific species of micro-organisms belonging to the same family or for the detection and/or the quantification of various isotypes of a general sequence belonging to a specific organism (including Single Nucleotide Polymorphism sequences)

### Background of the invention and state of the art

Biochips made with many specific capture nucleotide sequences are well suited tools to perform a discrimination between corresponding various homologous nucleotide sequences to be detected in a sample.

However, it exists differences in one or between microorganisms which differ by only one base of the DNA or RNA sequence. As these sequences are very similar, a cross-reaction may occur between one given target nucleotide sequence and different capture nucleotide sequences on which it may hybridise.

The biochips technology which allows by miniaturisation, the hybridisation and detection of one or more target nucleotide sequences are useful tools for the detection (discrimination) between one or more target nucleotide sequences, given to the high number of capture nucleotide sequences that are bound on the biochip. It allows also the determination of a gene expression pattern of a tissue, which is obtained by copying mRNA into cDNA and by their hybridisation upon biochips containing capture nucleotide sequences complementary to these sequences. As the cDNA sequences are different from each other, the level of cross-reaction is low, the capture nucleotide sequences being constructed from a bank of cDNAs cloned from the tissue (which are rather long sequences) and the rate and the yield of hybridisation is high.

When small capture nucleotide sequences are used the level of hybridisation is rather low, except if the target sequences are cut into smaller pieces. In solution, the rate of hybridisation is proportional to the square root of the strand length, the small one being the limiting one (Wetmur, J.G., Biopolymers 10, 601 (1971); Anderson et Young in "Nucleic acid and hybridisation", IRL Press, Hames, B. and Higgins, S. Editeurs, 73-111, Oxford-Washington DC (1985)). When the reaction is performed on a solid surface, the influence of the length of capture nucleotide sequences is even greater than in solution, so that the difference between the rate of fixation of a given target DNA sequence on small or long capture nucleotide sequences is very large.

The detection of DNA target nucleotide sequences itself is even more problematic since the DNA has first to be amplified (usually by PCR). Amplicons are double stranded DNA and tend to reassociate in solution rather than hybridise on capture nucleotide sequences, that are bound upon the solid surface (WO98/11253). This effect is also directly dependent on the length of the capture nucleotide sequences and small capture nucleotide sequences lead to a lack of sensitivity. It is possible to cut the DNA into pieces, in a non-specific way (given the large number of sequences to be analysed in chips). It is also possible to bind as much as possible capture nucleotide sequences upon a surface of the solid support in order to influence the yield of the reaction, but there are limitations to the concentration that can be bound. Finally the choice of the sequences is important, since even with the same length some sequences give higher hybridisation yield than other ones (formation of a secondary structure, which can favour or accelerate the first step leading to the double strand formation.

Therefore, capture nucleotide sequences of medium length have been proposed as a compromise which still gives a good yield of hybridisation while allowing the detection of target homologous sequences.

However, small capture nucleotide sequences allow discrimination between sequences which differ by as little as one base. One application of these array bearing small capture nucleotide sequences is the identification of Single Nucleotide Polymorphism (SNPs) sequences (WO98/56954).
The patent application WO93/25563 describes a method for discriminating between sequences which differ from each other by as little as a single nucleotide by using a OCR amplification method with special primer design. The sequence of the primer is composed of two portions, the 3' portion is specific for the desired target nucleic acid sequence and the 5' portion is complementary to a preselected nucleic acid sequence which is preferably bound to a solid support as one member of a grid having a group of sequences. Hybridization of the primer to its target sequence is followed by an extension step. Extension of the 3' portion of the primer with a labelled deoxynucleosides triphosphate yields a labelled extension product if, but only if, the template includes the correct target sequence. The labelled extension product is detected by hybridization of the 5' portion of the preselected sequence and not by a sequence portion specific of the target.
A method for detection of the presence or not of locus into genomic DNA has been proposed by Armour et al (Nucl.Ac. Res. 28, 605-609, 2000) based on the hybridization of medium probes on immobized long DNA. In this method, a genomic DNA is immobilized onto a nylon filter and then incubated with specific probes. The fixed probes have the same primer binding sites and are then amplified. The probes are of 140 to 600 bases and are not fitted to detect small difference in the genome like for the detection of homologous sequences.

### Aims of the invention

The present invention aims to provide a new and improved solution for the identification and/or the quantification of target nucleotide sequence among other possible homologous target sequences by using arrays bearing capture nucleotide sequences specific to the different homologous target sequences, and which does not present the drawbacks of the state of the art.

### Summary of the invention

The present invention allows the discrimination between homologous target sequences that could be present simultaneously in a sample, by an inversion of the hybridisation process of the sequences to be hybridised on the array.

The method according to the invention allows a identification (detection and characterisation) and/or a quantification of target nucleotide sequences based on a two-step binding process in which the nucleotide sequences that bind to the target in the first step are used in the second step as targets to be detected on an array.

In the method according to the invention, the target DNA (or RNA) sequences react first with different possibly labelled nucleotide sequences, then after washing said possibly labelled nucleotide sequences are detached from the target nucleotide sequences and finally identified by hybridisation on an array bearing capture nucleotide sequences complementary to the possibly labelled nucleotide sequences. According to the invention, it is the possibly labelled nucleotide sequences that hybridise on the array and not the target nucleotide sequences.

In the method according to the invention, the capture nucleotide sequences and the target nucleotide sequences have at least a part or a portion of their sequences which is identical, while the possibly labelled nucleotide sequences are complementary sequences to them. Such inverted method, as described in the enclosed claims, advantageously solves the problem of an efficient discrimination between homologous target sequences.

The present invention is related to a method for the identification and/or the quantification of one or more target nucleotide sequences being possibly present in a sample and for the discrimination from other homologous sequences, by using two sets of nucleotide sequences wherein in a first step, a first set of (possibly labelled) nucleotide sequences comprised between 8 and 60 bases, binds specifically to said target nucleotide sequences, wherein the (possibly labelled) sequences of the first set of nucleotide sequences which are not bound to the target nucleotide sequences are removed, wherein the (possibly labelled) nucleotide sequences of the first set of nucleotide sequences bound to the target nucleotide sequence are deshybridized from each other and are detected and/or quantified in a second step through hybridization with a second set of capture nucleotide sequences having at least a part of their nucleotide sequence complementary to the part of the nucleotide sequence of the (possibly labelled) sequences of the first set of nucleotide sequences which binds specifically to the target nucleotide sequences, said capture nucleotide sequences being immobilized upon the surface of a solid support according to an array of at least 4 discrete regions/cm², each of said discrete regions being bonded with one species of capture nucleotide sequences, and wherein the identification and quantification of the binding between the said (possibly labelled) nucleotide sequence and their corresponding capture nucleotide sequence is correlated with the identification and the quantification of said target nucleotide sequences present in the sample.

The present invention is also related to a diagnostic and/or quantification apparatus of one or more homologous
target nucleotide sequences possibly present in a sample, which comprises two separate chambers present upon a solid support, and a liquid connexion between the two chambers and wherein the second chamber contains capture nucleotide sequences immobilized to the surface of the solid support according to an array of at least 4 discrete regions/cm², each of said discrete regions being bound with one species of capture nucleotide sequences.

### Definitions

"Homologous nucleotide sequences" mean DNA or RNA sequences having the same nucleotide at corresponding positions. The degree of homology (or sequence identity) is calculated as the percentage of identical nucleotides at given locations after the sequences have been optimally aligned (full sequence) taking into account insertions or deletions like gaps in one of the two sequences to be compared. This is the case for sequences of a given gene, present in genetically different sources like different organism species or for proteins or enzymes having similar functions (from the same family or sharing common structural domain). The degree of homology (or sequence identity) can vary a lot with sequences being homologous at only one or more specific locations or all along their sequences. The parts (or portions) of the sequences which are identical in both sequences are said "conserved". The sequences showing a high degree of invariance in their sequences are said to be highly conserved and they present a high degree of homology. Sequences differing by only one base can be considered as the highest degree of homologous sequences. This is the case of sequences responsible for the Single Nucleotide Polymorphism sequences or SNP sequences.

Homology (or sequence identity) is calculated after alignment of sequences and are based on local homology algorithms which have been computerised (as for example but not limited to Clustal, Intelligenetics, Mountain View, California, or GAP, BESTFIT, FASTA and TFASTA in the Wisconsin Genetics Software Package, Genetics computer Group Madison, Wisconsin, USA or Boxshade).

### Short description of the drawings

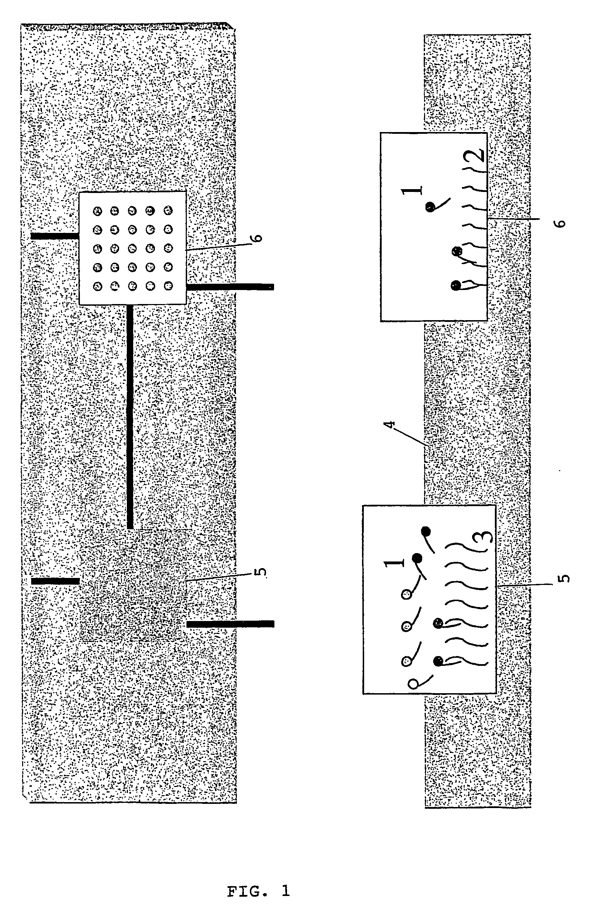

Figure 1 is a schematic presentation of reversed biochips according to the invention made of two chambers 5 and 6 present upon the same solid support 4 and connected for the purpose of automation.

### Detailed description of the present invention

The invention includes advantageously the use of double biochips wherein a first chamber 5 bearing a target nucleotide sequence 3 to be identified and isolated from a sample is introduced and a second chamber 6 comprising various bound capture nucleotide sequences 2 to the solid support 4 of the biochips 7, said capture nucleotide sequences being specific to the labelled nucleotide sequences 1, as represented in the Fig. 1. In order to make assays easy to perform, target nucleotide sequences 3 are immobilised on the solid support 4, so that they can react with various labelled nucleotide sequences 1 in the first chamber. However, this immobilisation is not essential to the invention since a first hybridisation can be performed in solution and the labelled nucleotide sequences 1 thereafter separated from the target nucleotide sequences 3 before being themselves detected on the array (hybridisation with corresponding capture nucleotide sequences 2).

The first advantage of the method is to ally a specificity with the rate of detection. The specificity is obtained by the fact that small labelled nucleotide sequences are used so that they can be chosen in the part of the target nucleotide sequences which differ from each other. The fact that it uses small sequences also allows to discriminate sequences differing by only one base (SNP sequences). In a preferred embodiment, the labelled nucleotide sequences have a sequence between 8 and 60 bases but preferably between 15 and 30 bases specific to the corresponding target nucleotide sequences.

The second advantage of the method according to the invention is that the hybridisation of the labelled nucleotide sequences 1 on the immobilised target nucleotide sequences 3 is a rather fast process compared to a situation where capture nucleotide sequences 2 are small and target nucleotide sequences 3 in solution are long fragments usually doubled stranded. Moreover, the rate can be accelerated by selecting a sequence for hybridisation of the labelled nucleotide sequences 1 located away from the binding site of the target nucleotide sequences 3 to the solid support and by adding the labelled nucleotide sequences 1 in excess. In a preferred embodiment, the labelled nucleotide sequences 1 are added in large excess, more than 100 times the target nucleotide sequences amount, in order to increase the rate and the yield of fixation of these labelled nucleotide sequences 1 on target nucleotide sequences 3.

All labelled nucleotide sequences 1 are present together for their hybridisation on target nucleotide sequences 3, which allows the reaction to be competitive between themselves. Since the sequences are present in the same or similar concentration, the yield of binding will depend on the affinity to the target nucleotide sequences. A favourable binding for the complementary sequence was observed compared to the non-complementary sequences even if they differ by as little as one base and discrimination between homologous sequences is very good.

In the second (array) chamber 6, the yield of hybridisation is similar for the various labelled nucleotide sequences 1 since they have the same (or similar) size and they hybridise on capture nucleotide sequences 2 of similar size. The intensity of the spots reflects the level of labelled nucleotide sequences 1 that have been detached from the first target nucleotide sequences (present in the sample) and are available for hybridisation in the second (array) chamber 6.

The second hybridisation of the sequences on the capture nucleotide sequences 2 in the array is a fast process since small sequences will react on capture nucleotide sequences 2 designed to allow fast reaction. The capture nucleotide sequences 2 can be optimised for such reaction to proceed at a fast rate, extending the specific sequence away from the binding point to the solid support 4 increases the rate of hybridisation. Since the complementary sequence is always in excess compared to the other non-complementary ones, the corresponding spot gives a higher signal from the beginning of the reaction, allowing to identify the target nucleotide sequence. If only qualitative determination is required, the reaction can be stopped before completion since the signal level on the spot will reflect their proportion in the solution from the start of the reaction.

One preferred embodiment of the method is the detection of double stranded target DNA. In the classical detection of target DNA sequences on array, the rate of hybridisation is low given the fact that the target DNA sequences can reform duplex in solution. One way to counteract such situation is to increase the length of the capture nucleotide sequences but in this case homologous sequences will cross-hybridised on the same capture nucleotide sequences, so leading to false detections. In this invention, the target is rendered single stranded either before or after being immobilised so as to reduce or remove the competition between the labelled capture nucleotide sequences and the second target strand for hybridisation. The labelled nucleotide sequences are preferably single stranded nucleotides in order to avoid their rehybridisation in solution.

The invention is particularly suitable for identification and/or quantification of homologous sequences (identification of a particular strain of microorganism in a sample or in research when genes have to be identified specifically among a population of related genes coding for proteins having different roles in normal or pathological situation). This is especially true for regulatory genes like receptors, kinases, phosphatases, cyclins, transcriptional factors or oncogenes. Having part of their sequences identical, target homologous nucleotide sequences can be amplified or copied by using consensus primers. In many applications, the role of the detection is to discriminate between target nucleotide sequences of similar organisms (homologous DNA or RNA sequences). In that case, parts or portions of the sequences that are conserved, are used for the binding of primers that recognise all these homologous nucleotide sequences (if present in the sample). The use of only one primer pair for one family or genus of organisms is an advantage compared to the use of primers specific for each species or subspecies since some families of organisms comprise more than thirty species. It is also difficult (if not impossible) to find amplification conditions which would allow the amplification of all these species in one multiplex PCR (a multiplex PCR for 5 different nucleotide sequences is already difficult to handle in a reproductive way on samples). According to the invention, an assay can be designed in order to amplify each family of organisms (like the bacteria families) with only one primer pair for each family. If only a copy is performed as in the case of mRNA retrotranscription, a single pol-dT can serve for the transcriptase to perform the copy into cDNA.

Preferably, small labelled nucleotide sequences 1 are used for hybridisation in the first as well as in the second hybridisation steps. The use of small nucleotide sequences 1 allows the discrimination of very homologous sequences (from 30 to 98% homology). If the labelled nucleotide sequences are appropriately chosen, they can discriminate between two sequences differing in one base (SNP) thus allowing the determination of polymorphism.

In a preferred embodiment, the labelled nucleotide sequences 1 contain one part of their sequence specific of their target nucleotide sequences 3 and an additional tail which is non specific of the target nucleotide sequences 3 and specific of a given labelled nucleotide sequence. This tail is complementary of the capture nucleotide sequences 2 present on the array chamber 6. Since the tail is different for each labelled nucleotide sequences 1, they stabilise perfect match on the array and a consequence is that closely related labelled nucleotide sequences differing for example in one base in the part of the sequence specific of the target nucleotide sequences will be better discriminated on the array.

The method can be applied for detection of SNP sequences by using modifications of the nucleotide sequences one hybridised on the target DNA sequence and then detected in a second step on the array chamber 6 bearing the various capture nucleotide sequences 2.

In one embodiment, the invention is applied to determination of SNP sequences by using a mixture of two or more labelled nucleotide sequences terminated by a different nucleotide which bind to the target nucleotide sequence adjacent to the possible SNP sequence, one of end nucleotide of the nucleotide sequence being perfectly matched to the SNP sequence. The presence of another annexed nucleotide sequence which will bind beside the labelled nucleotide sequence, allows the perfectly matched nucleotide sequence to be ligated to this nucleotide sequence by a ligase. The ligated nucleotide sequence is then detected on the second array so that the SNP sequence is identified. The various labelled nucleotide sequences may bear different labels such as Cy3, Cy5 or Cy7 so as to be easily identified. If not labelled then the second annexed nucleotide sequence has to be labelled so that the resulting ligated product is labelled.

In another embodiment of the invention, a mixture of the labelled nucleotide sequence is used, each one differing in at least one base located at the site on the same SNP. After hybridisation, the mismatch nucleotide sequences are cut by a nuclease specific for single strand but without effect on the double stranded DNA and the process repeated. The uncut and cut nucleotide sequences are then processed for hybridisation on the array chamber 6. In appropriated hybridisation conditions and with well chosen capture nucleotide sequences on the array chamber, they can either hybridise the long uncut or the small cut nucleotide sequence thus allowing identification of the SNP sequence.

In another embodiment of the invention, a mixture of several nucleotide sequences is used which recognised different parts of the target nucleotide sequences, like SNP sequences located along the target nucleotide sequence, and detected in the second step of the method on the array. Numerous capture nucleotide sequences 2 can be fixed to the array and be able to discriminate between numerous labelled nucleotide sequences 1. In this way, it is possible to investigate in one assay for multiple variations like mutations possibly present in a given target nucleotide sequence. This is especially advantageous when all these variations are potentially present in the target and if they are correlated with a pathological situation. This is the case for mutations occuring in oncogenes (like P53); many of them being associated with cancer progression, in HIV virus enzymes leading to resistance to drug treatment. SNP are also probably linked to individual variability influencing the response to drug treatment or to pathologies.

In a preferred embodiment, several target nucleotide sequences are used in the first hybridisation step in order to obtain a broader response to the given problem and taking profit of the numerous detections possible on the array.

Advantageously, target nucleotide sequences can be detected without being labelled by a copy or amplification step, thus making the direct detection of DNA or RNA sequences possible (in most previous methods, target nucleotide sequences are labelled during the amplification or transcription step). This allows a simplification in the final use of the biochips, since the user can perform the amplification in any way and does not have to bother with the possible interferences of labelled reagent during this step. This property also allows the identification of sequences directly after extraction from samples, removing the step of copying or amplification making it easier to perform. This direct detection can only be performed, when enough material is available. The applications according to the invention apply (but are not limited) to the RNAs like the ribosomal RNA: 16s, 23s, 18s or 25s but also the mRNA. Since these RNA are single strand nucleotides, it is not necessary to use denaturation as drastic as in the case of double stranded amplicons after their immobilisation on the support so that non covalent binding can be used in this step. Detection of RNA, ribosomal or messenger, either directly or after their copy into their complementary DNA sequence is a preferred embodiment of this invention since they are usually present in multiple copies in cells so that in many applications, their detection can be performed without amplification. This direct detection also facilitates the quantification since avoiding the amplification reduced the variation due to this step which is very often difficult to master. Direct detection can also be adapted to long DNA sequences after their extraction and denaturation or after their cutting into smaller pieces.

In a preferred embodiment of the invention, the target nucleotide sequence 3 is amplified (PCR) by using consensus primers (sequences which will be amplified by the same primers). Homologous sequences are for example sequences of genes present in the same family or genus of microorganisms. For this amplification, one of the primer will be aminated so that after amplification, one of the amplicon strand will bear this amino group. The amplicons are then incubated on a surface bearing aldehyde groups. The reaction between the amine and the aldehyde is spontaneous in normal conditions, no other reagent are necessary. After the reaction a reducing agent is then added (NaBH₄) in order to eliminated the reactive double bonds. The second strand is then remove by using denaturation conditions for the nucleotide sequences. It is possible to increase the temperature above the melting temperature of the nucleotide sequences, so that they separate from the target strand, but other conditions like the use of alkaline solution may be used (a concentration of 0.1 or even 0.05 N of a NaOH solution is sufficient to separate the two strands).

Binding of the amplicons to the activated surface can be limited by the presence of the primers which are still present in the solution after the amplification. These primers can also react on the activated surface thus lowering the fixation of the target amplicons. When necessary, one separates the amplicons from the primers before performing their fixation on the surface (separation on spin column, but silica derived adsorption like the Nucleo trap PCR purification kit (Clonetech) or the filtration on gel filter (Nucleo Spin Clonetech) are also well suitable for such separation).

Separations of DNA fragments according to their size are also well obtained using electrophoretic methods and the miniaturisation of such techniques allows them to be now suitable for current applications like necessary in such routine applications.

According to the invention, the assay is a tandem chips performed on the same support 4. On the first chamber 5, the target nucleotide sequences are immobilised by covalent fixation on an activated support 4. Covalent reactions are well known in chemistry and many of them are already used for chips formation, for example by the reaction of an aminated target nucleotide sequence with an aldehyde group present on the chips surface. In another embodiment, the target sequences are biotinylated either directly or during the copy or amplification step preferably on one strand and the target immobilised on streptavidin coated surface. In another embodiment, any hapten or ligand is fixed on the target sequence so that it is immobilised by the corresponding antibody or receptor immobilised on a surface.

Many supports can be adapted for this step to occur. Glass is activated so as to bear aldehyde groups. There are many different ways to graft aldehyde groups on a surface. Glass reacts with aminosilane and then with glutaraldehyde. One may bind carboxyl groups upon the surface of the solid support and then reduce them into aldehyde. Alcohol functions can also be oxidised into aldehydes. However the use of bifunctional activators such as SMCC or DSS are also useful since they can activate amino groups and link them respectively on thiol or amine groups. Such chemical coupling gives a covalent fixation of the target nucleotide sequences to the support especially useful when the target nucleotide sequences are double stranded and have to be treated in a denaturation solution. However simple adsorption on surface, like nylon, cellulose derivative or positively charge surfaces for example covered with polylysine or on copolymer like the poly (phelys) is also a method suitable for the adsorption of nucleotide sequences on a surface. In this case, conditions are worked out for the adsorption of long target DNA fragments rather than small primers.

If glass has many advantages like being inert and having a low auto-fluorescence, other supports especially polymers can be useful since they can also be obtained with various chemically well defined groups at their surface, thus making the fixation of the target DNA possible. Polystyrene has been activated so as to obtain carboxyl or amino groups on the surface thus allowing covalent fixation of amino-DNA (Anal. Biochem. 236, 85 (1996). Most of polymers can be activated in order to obtain carboxyl, amino or aldehyde groups. Polystyrene is available not only as flat support but also as microbeads which are advantageously used to perform the first binding step of the labelled probes on the immobilised targets.

All other supports which can fix DNA can be used, like filters, silicon supports, metallic supports, compact-discs, plastic or electronic devices. Advantageously, said solid support is a single glass or plastic plate which may comprise additional means (barcodes, markers, etc.) or media (coating, etc.) for improving the method according to the invention. A combination of supports like glass inserted into plastic support is useful for handling the chips for automation.

After and/or before binding, the target nucleotide sequences are denatured, leading to the formation of a single stranded immobilised target nucleotide sequences. The solution containing the various labelled nucleotide sequences are then added to this first monochips and they can react if their complementary sequence is present on the target nucleotide sequences. After washing, the solution is heated and the bound nucleotide sequences detached from the target nucleotide sequences and are found in solution. This solution is then transferred to the second chamber 6 by specific channels or other means (washing step with the use of micropumps or valves) that contains the capture nucleotide sequences specific for the labelled nucleotide sequences. The second hybridisation is then performed which will allows to identify the labelled nucleotide sequences and thus the target nucleotide sequence. If the different steps are well performed the assay can be made quantitative thus allowing not only the identification but also the quantification of target nucleotide sequences. As all steps are performed on the same surface 4, the assay is easy to perform for the user and allows an automation.

In the preferred embodiment of this invention, consensus primers are used in PCR amplification of the homologous sequences possibly present in the sample. Functional groups such are NH₂ or biotin are incorporated so as to attach them to a solid surface as described here above. Other amplification methods like LCR, CPR, NASBA, ICR, TMA or avalanche DNA are also possible. RNA amplification can be performed in the same way after a retrotranscription. Short sequences of DNA can also be obtained by specific cleavage with restriction enzymes and small sequences of RNA by non specific cleavage by heat or in the presence of base (WO97/10365).

Indirect labelling by using biotinylated labelled nucleotide sequences which will thereafter bind a streptavidin-conjugate either with fluorescent molecules or with enzyme or with colloidal gold particles, may be used. These gold particles can be detected by themselves or serve as catalysts for the reduction of silver. The precipitate is then detected and possibly quantified (EP-99870106.4).

Labelling could be also obtained with hapten and antibody reaction, the antibody being label or conjugate to molecule which can give a signal.

All methods of detection for DNA based on fluorescent, colorimetric, magnetic, electronic, electroluminescence, bioluminescence or radioactivity adapted for DNA chips can be used for the detection of the array.

The selection of the detection labelled nucleotide sequences and their complementary capture nucleotide sequences is a crucial step in the overall process when homologous sequences have to be specifically differentiated from each other. Higher are the homologies between the sequences, more important is the selection of these sequences. Preferably, one selects a small sequence region (or portion) where the homologous sequences differ most (or a lot) and to design the labelled nucleotide sequences as complementary to this region (or portion). The length of the sequences will also vary according to the homology, being longer when the sequences are rather different and smaller when they are very homologous. The sequences will be between 15 and 30 bases but as small as 8 bases and as long as 60 bases can be used. The capture nucleotide sequences that are spotted on the array are complementary to the labelled nucleotide sequences. In one preferred embodiment, these sequences 2 are located at a certain distance from the surface 4 either by having a non specific sequences which is bound to the surface 4 or by using a linker for the fixation.

The use of a single surface in order to perform the two hybridisation steps allows the process to be adapted for automation and for microfluidic technologies. The surface is divided in two chambers, the first one where the target DNA or RNA will be bound and where the labelled sequences will hybridise on the target sequences, and the second one which is the array and contains the various capture sequences. Solutions can be injected and removed from each chamber by channels and pumps so that incubations and washing are possible. The chambers can also be heated at the required temperatures.

The two chambers can also be connected by a small pipe or channel so that after detachment of the labelled nucleotide sequences from the target, the solution can be transferred into the second chamber and process for hybridisation on the array. The overall process including even the detection can be automated thus making this reversed chips detection an easy process. This invention repeats the steps of binding and detachment of labelled nucleotide sequences on the immobilised targets so that the specific nucleotide sequences accumulated in the array for their discrimination on capture nucleotide sequences. Automation of the process by automate or machine is particularly well suited for repeating the process.

This technology can be easily adapted to existing automates. One of them is the VIDAS (Biomerieux, Lyon, France), developed for ELISA assay. It has the particularity to perform the antigen/antibody reaction in the tibs which is then passed step by step to the various solutions for washing and incubation. The final signal obtain in this tip is then recorded. The array can be inserted into a device like a dispensary tip so that solutions is pump inside and is made to react, wash and incubate on the array. Performing the reaction in a tip has been developed for antibody-antigen reaction and is well adapted to automatisation as performed by the VIDAS machine (Biomerieux, Lyon, France). After the reaction is complete the signal is detected on the array as described here under.

Double array system could be treated in the same way, each one being inserted into a micropipette device and being connected to each other for passing the solution of nucleotide sequences when required by the protocol.

Microfluidic techniques make also possible the overall procedure of amplification and/or the separation of amplicons from the excess primers to be performed on the same surface, thus leading to a "lab on a chips" product. The final surface will contain several or all of the compartments: a chamber for the PCR to be conducted, a separation device for the purification of the amplicons if necessary, a chamber for the immobilisation of the amplicons and the first hybridisation with the various labelled nucleotide sequences and finally a chamber with the array for the final identification of the detection nucleotide sequences. The overall product would bear the necessary connections for the liquid to be conducted between the chambers and for the incubations and washings with the external solutions. A detector, could also be added to the automate in order to obtain a fully automated analysis of the assay. Separation of the amplicons from the primers can be done using filtration, adsorption-desorption process or micro electrophoresis as proposed for example by ACLARA Biosciences, (Mountain View, Ca, USA).

### Analysis of the result of the hybridisation

The result of the hybridisation on the array has to be detected and/or quantify by machine using detection system adapted to the signal which is to be emitted by positive hybridisation. The most common labelling of DNA used in micro-array is based on the use of fluorescent nucleotide sequences. In this case fluorescent scanners serve as detectors (i.e. confocal fluorescent scanner (Genetic Microsystem, Woburn, MA, USA)).

The formation of a precipitate on the positive spots, especially silver precipitate after labelling with nano-gold, is especially suitable for easy detection since colorimetric scanners or photographic detectors which are particularly low cost technology can detect and/or quantify the level of reaction (EP-99870106.4).

A software analysis for image recognition has been adapted to the spots, followed by a quantification of the signal present on each spot which take into account the level of the background. Another type of analysis is based on correlation analysis of the spots which takes into account the shape and the level of each pixel of the spot. A classification of the spots according to the level of correlation allows then a classification into positive or negative spots, giving the user an easy answer for the presence or not of the targets in the original sample.

Quantification of the hybridisation is performed using conventional approaches either by internal or external standard which allows corrections for the different yields obtained at the various steps of the process starting with the extraction, the copy or amplification, the first hybridisation and the second hybridisation on the array. In the preferred embodiment, an external standard at known concentration is added in the sample and process through all steps in order to correct for the efficiency of the overall process. Since the method usually contains a copy or amplification method, a competitive standard is the preferred embodiment of the invention. One of them has been proposed in the document WO98/11253.

Preferably, the hybridisation yield of the standard through this specific sequence is identical or differ no more than 20% from the hybridisation yield of the target sequence and quantification is obtained as described in WO98/11253.

Said standard nucleotide sequence, external and/or internal standard, is also advantageously included in the kit (device) or apparatus according to the invention, possibly with all the media and means necessary for performing the different steps according to the invention (hybridisation and culture media, polymerase and other enzymes, standard sequence(s), labelling molecule(s), etc.).

### Example

### Identification of Staphylococcus species by detection of the specific femA genes

The FemA genes are very conserved genes and they have an homology of 50 to 90 % between the different Staphylococcus species. The identification of *Staphylococcus epidermidis* in a sample was performed by using a preamplification by consensus primers which can amplify the 5 most common Staphylococcus: *S. aureus, S. epidermidis, S. haemolyticus, S. hominis,* and *S. saprophiticus.* One of the primer was aminated, so that one stand of the amplicons bear an amino group at its 5' end. The amplicons were then covalently fixed to a glass bearing aldehyde groups and then denatured by heating at 100 °C. The labelled nucleotide sequences specific of the 5 Staphylococcus species were then incubated with these single stranded amplicons. After reaction and washing, there were recovered in a solution of NaOH. This solution was then added on the array formed by spots of capture nucleotide sequences specific for each of the labelled nucleotide sequences. After reaction and washing, a streptavidin-Cy5 conjugate was added followed by the reading using a confocal fluorescent scanner (Genetic Microsystem). The principle of this method is shown in Fig. 1 and Fig. 2. Only the spots bearing the capture nucleotide sequences specific for *S. epidermidis* are positive.

### Amplification of the FemA gene using consensus primers

The sequences of the primers used for PCR amplification are the following: FP 1 : 5′ CCA CTA GCG TAC ATC AAT TTT GA 3′ FP 1HS : 5′ CCC ACT CGC TTA TAT AGA ATT TGA 3′ FP 3 : 5′ GGT TTA ATA AAG TCA CCA ACA TAT T 3′ with a NH₂ group at 5' end? These primers amplify a sequence of 487 bp within the femA gene.

### Fixation of the amplicons DNA on a surface

Amplicons are purified by chromatography on High Pure PCR Product Purification kit (Boehringer, Mannaheim, Germany) in order to eliminate aminated primers and free nucleotides. For the fixation on the surface, 100 µl of the solution SSC3X pH 5 containing 150 nM of purified amplicons were incubated for 30 min at 23°C on silylated microscopic slides (Cell associates, Houston, USA) framed by an hybridisation chamber. After incubation, the slides were washed once with 0.1% SDS, twice with water, and then incubated 5 min with NaBH₄ (2.5 mg dissolved in 750 £1 of PBS and 250 µl of 100% ethanol) and 3 min in boiling water slides were stored at 4°C until used.

### Hybridisation and removal of the specific nucleotide sequences from the target

The specific biotinylated nucleotide sequences for the 5 staphylococci species detection are the following :
*S. aureus* ASaur01 : 5′ CTA AAT GAA GAG CGT GAT ATT TTA AAT 3′
*S. epidermidis* ASepi01 : 5′ CTA AAT AAT GAA AGA AAT GTG CTT AAT 3′
*S. haemolyticus* AShae01 : 5' TTA CAA AAT GAA CGT GAA ACT TTA AAT 3'
*S. hominis* AShom01 : 5' CTT CAT GCA GAA CGT CAG ACA TTA AAT 3'
S. saprophyticus

ASsap01 : 5' TTA AAG GCT GAA CGC GAA GTA TTA AGT 3'

The sequence of the biotinylated nucleotide sequence control is :

CMV23Biot 5' GGT TAT CAG AGG CCG CTT GGC CA 3'

Each probe bears a biotin at its 5' end.

For the hybridisation, 100 µl of hybridisation solution are loaded on glass slide bearing the target DNA. This mixture contains 0.5 M phosphate buffer pH 7.4, 7% SDS, 100 £g/ml salmon sperm DNA, 30 nM of *S. epidermidis* biotinylated nucleotide sequences (1 nucleotide sequence) or 30 nM of each of the nucleotide sequences (5 nucleotide sequences). Hybridisation is carried out in an hybridisation chamber at 60°C for 2h. Samples are washed 4 times with Maleic buffer 10 mM pH 7.5, NaCl 15 mM, Tween 0.03%.

Probes hybridised are released with the incubation for 5 min at 25°C with 70 µl of NaOH 0.05 N within the hybridised area delimited by an hydrophobic pen. 50 µl of solution is then transferred into a microtube and neutralised with 56 µl of solution containing 0.7 M phosphate buffer pH 7.4, 10% SDS, 200 µg/ml salmon sperm DNA, 10 nM biotinylated CMV nucleotide sequence (hybridisation control).

### Detection of the labelled nucleotide sequences on the array

### Sequences of the capture nucleotide sequences

The sequences of the capture nucleotide sequences for the 5 staphylococci are the following:
*S. aureus* ATaur02 : 5′ ATT TAA AAT ATC ACG CTC TTC ATT TAG 3′
*S. epidermidis* ATepi02 : 5′ ATT AAG CAC ATT TCT TTC ATT ATT TAG 3′
*S. haemolyticus* AThae02 : 5′ ATT TAA AGT TTC ACG TTC ATT TTG TAA 3′
*S. hominis* ATthom02 : 5′ ATT TAA TGT CTG ACG TTC TGC ATG AAG 3′
*S. saprophyticus*

ATsap02 : 5′ ACT TAA TAC TTC GCG TTC AGC CTT TAA 3′

The sequence of the capture nucleotide sequence control is :

CMVNH2 : 5' TGG CCA AGC GGC CTC TGA TAA CC 3'

Each capture nucleotide sequence bears a NH₂ group at its 5' end.

### Capture probe immobilisation

The protocol described by Schena et al (Proc. Natl. Acad. Sci. USA 93, 10614, 1996) for the grafting of aminated DNA to aldehyde was followed with minor changes.

The aminated capture nucleotide sequences were spotted at a concentration of 1.6 µM. The capture nucleotide sequences were printed onto the silylated microscopic slides with a home made arrayer. The capture nucleotide sequences were complementary to the labelled nucleotide sequences and were terminated by an amino group at their 5' end. 250 µm pins from Genetix (UK) and silylated (aldehyde) microscope slides from Cell Associates (Houston, USA) were used. The spots have 400 µm in diameter and the volume dispensed I about 1 nanoliter. The slides were dried at room temperature and stored at 4°C until used.

### Hybridisation and detection

106 µl of denatured product are incubated in hybridisation chamber for 30 min at 50°C. Slides are then washed 4 times with Maleic buffer 10 mM pH 7.5, NaCl 15 mM, Tween 0.03% (washing buffer). Glass samples are incubated 45 min at room temperature with 800 µl of streptavidin labelled cyabin5 (Sigma St Louis, Mi) 500x diluted in Maleic buffer 100 mM pH 7.5, NaCl 150 mM, Gloria milk powder 0.1%. Slides are washed 5 times with washing buffer, rinced ionce with water and dried. Slides are read with a confocal scanner from Genetic Microsystem (Woburn, MA, USA).

Table 1 gives a result obtained with reversed biochips according to the invention for the detection of a specific sequence of *Staphylococcus epidermidis* (A) when target *S. epidermidis* amplicons were immobilised and incubated in a first chamber 5 with a mixture of the labelled nucleotide sequences 1 specific of the 5 *Staphylococcus* strains or (B) when target *S. aureus* amplicons were immobilised and incubated with specific labelled *S. epidermidis* nucleotide sequences. The nucleotide sequences 1 detached from the target nucleotide sequence after hybridisation were then transferred on an array (chamber 6) bearing capture probes 2, for the 5 possible labelled probes specific of the 5 Staphylococcus species sequences and detected.

**Table I**

| Detection on the array bearing the following capture probes Step II | A. Step I Immobilised target sequence of *S. epidermidis* in the presence of the 5 labelled probes | B. Step I Immobilised target sequence of *S. aureus* in the presence of the *S. epidermidis* labelled probe |
|---|---|---|
| *S. aureus* | 2 | 0 |
| *S. epidermidis* | 19 | 0 |
| *S. haemolyticus* | 1 | 1 |
| *S. hominis* | 0 | 0 |
| *S. saprophyticus* | 0 | 0 |
| Control hybridization + | 51 | 25 |
| Control hybridization - | 1 | 0 |

## Claims

1. Method for an identification and/or a quantification of one or more target nucleotide sequences (3) being possibly present in a sample and for a discrimination from other homologous sequences, by using two sets of nucleotide sequences (1 and 2),
- wherein in a first step, a first set of (possibly labelled) nucleotide sequences (1) comprised between 8 and 60 bases, binds specifically to said target nucleotide sequences (3),
- wherein the (possibly labelled) sequences of the first set of nucleotide sequences (1) which are not bound to the target nucleotide sequences (3) are removed,
- wherein the (possibly labelled) nucleotide sequences (1) of the first set of nucleotide sequences bound to the target nucleotide sequence (3) are deshybridized from each other and are detected and/or quantified in a second step through hybridization with a second set of capture nucleotide sequences (2) having at least a part of their nucleotide sequence complementary to the part of the nucleotide sequence of the (possibly labelled) sequences (1) of the first set of nucleotide sequences which binds specifically to the target nucleotide sequences (3), said capture nucleotide sequences (2) being immobilized upon the surface (4) of a solid support according to an array of at least 4 discrete regions/cm², each of said discrete regions being bonded with one species of capture nucleotide sequences (2), and
- wherein the identification and quantification of the binding between the said (possibly labelled) nucleotide sequence (1) and their corresponding capture nucleotide sequence (2) is correlated with the identification and the quantification of said target nucleotide sequences (3) present in the sample.

2. The method according to claim 1, wherein the target nucleotide sequences (3) are firstly bound upon a solid support (4), preferably by covalent binding, before their hybridization binding with one or more (possibly labelled) sequences (1) of the first set of nucleotide sequences.

3. The method according to claim 1 or 2, wherein the binding between the target nucleotide sequences (3) and the (possibly labelled) sequences (1) of the first set of nucleotide sequences occurs in solution.

4. The method according to any of the preceding claims 1 to 3, wherein the (possibly labelled) sequences (1) of the first set of nucleotide sequences have a length which is essentially identical or similar to the length of the capture nucleotide sequences (2) of the second set of nucleotide sequences.

5. The method according to any of the preceding claims, wherein the target nucleotide sequences (3) to be identified and/or quantified present a homology with other homologous sequences present in the sample which is higher than 30%, preferably higher than 60%, more preferably higher than 80%.

6. The method according to any of the preceding claims, wherein the target nucleotide sequences (3) to be identified and/or quantified differ from homologous sequences by only one base.

7. The method according to the claim 1, wherein the capture nucleotide sequences (2) of the second set of nucleotide sequences have a sequence which is complementary to the (possibly labelled) nucleotide sequences (1) of the first set of nucleotide sequences upon their complete sequence or have a sequence which is bound to the solid support through a spacer having a length comprised between 10 and 200 bases.

8. The method according to any of the preceding claims, wherein the (possibly labelled) nucleotide sequences (1) contain a mixture of two or more labelled sequences terminated by a different nucleotide which binds to the target nucleotide sequence adjacent to a possible SNP, one of the end nucleotide being perfectly matched to the SNP, and another annexed probe which will bind to the other site of the SNP target sequence, the perfectly matched probe being ligated to this sequence by a ligase before detected on a corresponding capture nucleotide sequence (2).

9. The method according to any of the preceding claims, wherein the labelled nucleotide sequences (1) contain a mixture of probes, each one differing in at least one base located at a SNP site and treated with a specific nuclease before being detected on a corresponding capture nucleotide sequence.

10. The method according to any of the preceding claims, wherein the one or more target nucleotide sequences (3) to be detected and/or quantified are rRNAs, preferably selected from the group consisting of 16S, 23S, 18S and 25S rRNAs.

11. The method according to any of the preceding claims 1 to 9, wherein the one or more target nucleotide sequences (3) to be detected and/or quantified are mRNAs, preferably a mRNA retrotranscripted into a cDNA by a consensus sequence.

12. The method according to any of the preceding claims, wherein the solid support is selected from the group consisting of glass, electronic device, silicon support, plastic support, compact disc, filter, metallic support, polylysine coated surfaces or a mixture thereof.

13. The method according to any of the preceding claims, wherein the first set of (possibly labelled nucleotide sequences (1) binds specifically to the target nucleotide sequences (3) in a first chamber (5),
- wherein the (possibly labelled) sequences of the first set of nucleotide sequences (1) which are bound to the target nucleotide sequences (3) are removed from the first chamber (5) and transferred to the second chamber (6), and wherein the possibly labelled nucleotide sequences (1) of the first set of nucleotide sequences are detected and/or quantified through hybridisation with the second set of capture nucleotide sequences (2) in the second chamber (6).

14. A diagnostic and/or quantification apparatus of one or more homologous target nucleotide sequences possibly present in a sample, which comprises two separate chambers (5 and 6) present upon a solid support (4), and a liquid connexion between the two chambers (5,6) and wherein the second chamber (6) contains capture nucleotide sequences (2) immobilized to the surface of the solid support according to an array of at least 4 discrete regions/cm², each of said discrete regions being bound with one species of capture nucleotide sequences (2).

15. A diagnostic and/or quantification apparatus according to the claim 14, which further comprises a first set of (possibly labelled) nucleotide sequences (1) able to react with the target nucleotide sequences to be detected and/or quantified in the first chamber (5) and being able to hybridise the capture nucleotide sequences (2) present in the second chamber (6).

16. The apparatus of claim 14 or 15, wherein the two chambers (5 and 6) are present on a single surface of a solid support (4).

17. The apparatus according to any of the preceding claims 14 to 16, wherein the two chambers (5 and 6) are connected by a small pipe or channel for solution transfer from one chamber (5) to the other chamber (6).

18. A machine or automate for performing the various steps of the method according to any of the preceding claims 1 to 13 and comprising the apparatus according to the claims 14 to 17.

## Patentansprüche

1. Verfahren für eine Identifizierung und/oder eine Quantifizierung einer oder mehrerer Nukleotidzielsequenzen (3), die möglicherweise in einer Probe vorhanden sind, und für eine Unterscheidung von anderen homologen Sequenzen durch Verwendung von zwei Sätzen von Nukleotidsequenzen (1 und 2),
- wobei in einem ersten Schritt ein erster Satz von (gegebenenfalls markierten) Nukleotidsequenzen (1), die zwischen 8 und 60 Basen umfassen, spezifisch an die Nukleotidzielsequenzen (3) bindet,
- wobei die (gegebenenfalls markierten) Nukleotidsequenzen des ersten Satzes von Nukleotidsequenzen (1), die nicht an die Nukleotidzielsequenzen (3) gebunden sind, entfernt werden,
- wobei die (gegebenenfalls markierten) Nukleotidsequenzen (1) des ersten Satzes von Nukleotidsequenzen, die an die Nukleotidzielsequenz (3) gebunden sind, voneinander enthybridisiert werden und in einem zweiten Schritt durch Hybridisierung mit einem zweiten Satz von Capture-Nukleotidsequenzen (2) nachgewiesen und/oder quantifiziert werden, bei denen mindestens ein Teil ihrer Nukleotidsequenz zu dem Teil der Nukleotidsequenz der (gegebenenfalls markierten) Sequenzen (1) des ersten Satzes von Nukleotidsequenzen, welcher spezifisch an die Nukleotidzielsequenzen (3) bindet, komplementär ist, wobei die Capture-Nukleotidsequenzen (2) auf der Oberfläche (4) eines festen Trägers gemäß einer Anordnung von mindestens 4 diskreten Bereichen/cm² immobilisiert sind, wobei an jeden der diskreten Bereiche eine Art von Capture-Nukleotidsequenzen (2) gebunden ist, und
- wobei die Identifizierung und Quantifizierung der Bindung zwischen den (gegebenenfalls markierten) Nukleotidsequenzen (1) und ihren entsprechenden Capture-Nukleotidsequenzen (2) mit der Identifizierung und der Quantifizierung der in der Probe vorhandenen Nukleotidzielsequenzen (3) korreliert.

2. Verfahren nach Anspruch 1, wobei die Nukleotidzielsequenzen (3) vor ihrer Hybridisierungsbindung mit einer oder mehreren (gegebenenfalls markierten) Sequenzen (1) des ersten Satzes von Nukleotidsequenzen zuerst auf einem festen Träger (4) gebunden werden, vorzugsweise durch kovalente Bindung.

3. Verfahren nach Anspruch 1 oder 2, wobei die Bindung zwischen den Nukleotidzielsequenzen (3) und den (gegebenenfalls markierten) Sequenzen (1) des ersten Satzes von Nukleotidsequenzen in Lösung abläuft.

4. Verfahren nach irgendeinem der vorherigen Ansprüche 1 bis 3, wobei die (gegebenenfalls markierten) Sequenzen (1) des ersten Satzes von Nukleotidsequenzen eine Länge haben, die im Wesentlichen identisch oder ähnlich ist wie die Länge der Capture-Nukleotidsequenzen (2) des zweiten Satzes von Nukleotidsequenzen.

5. Verfahren nach irgendeinem der vorherigen Ansprüche, wobei die zu identifizierenden und/oder zu quantifizierenden Nukleotidzielsequenzen (3) eine Homologie mit anderen in der Probe vorhandenen homologen Sequenzen darstellen, die höher als 30 %, vorzugsweise höher als 60 %, mehr bevorzugt höher als 80 % ist.

6. Verfahren nach irgendeinem der vorherigen Ansprüche, wobei sich die zu identifizierenden und/oder zu quantifizierenden Nukleotidzielsequenzen (3) von homologen Sequenzen um nur eine Base unterscheiden.

7. Verfahren nach Anspruch 1, wobei die Capture-Nukleotidsequenzen (2) des zweiten Satzes von Nukleotidsequenzen eine Sequenz aufweisen, die zu den (gegebenenfalls markierten) Nukleotidsequenzen (1) des ersten Satzes von Nukleotidsequenzen auf ihrer vollständigen Sequenz komplementär ist, oder eine Sequenz aufweisen, welche über einen Abstandshalter mit einer Länge, die zwischen 10 und 200 Basen umfasst, an den festen Träger gebunden ist.

8. Verfahren nach irgendeinem der vorherigen Ansprüche, wobei die (gegebenenfalls markierten) Nukleotidsequenzen (1) eine Mischung aus zwei oder mehr markierten Sequenzen enthalten, die von einem unterschiedlichen Nukleotid beendet werden, welches an die neben einem möglichen SNP liegende Nukleotidzielsequenz bindet, wobei eines der Endnukleotide perfekt mit dem SNP übereinstimmt, und eine andere angehängte Sonde, welche an die andere Stelle der SNP-Zielsequenz bindet, wobei die perfekt übereinstimmende Sonde durch eine Ligase an diese Sequenz ligiert wird, bevor sie auf einer entsprechenden Capture-Nukleotidsequenz (2) nachgewiesen wird.

9. Verfahren nach irgendeinem der vorherigen Ansprüche, wobei die markierten Nukleotidsequenzen (1) eine Mischung von Sonden enthalten, die sich jeweils in mindestens einer sich an einer SNP-Stelle befindenden Base unterscheiden und mit einer spezifischen Nuklease behandelt werden, bevor sie auf einer entsprechenden Capture-Nukleotidsequenz nachgewiesen werden.

10. Verfahren nach irgendeinem der vorherigen Ansprüche, wobei die eine oder mehrere nachzuweisenden und/oder zu quantifizierenden Nukleocidzielsequenzen (3) rRNAs sind, die vorzugsweise aus der Gruppe ausgewählt sind, die aus 16S, 23S, 18S und 25S rRNAs besteht.

11. Verfahren nach irgendeinem der vorherigen Ansprüche 1 bis 9, wobei die eine oder mehrere nachzuweisenden und/oder zu quantifizierenden Nukleotidzielsequenzen (3) mRNAs sind, vorzugsweise eine mRNA, die von einer Konsensussequenz in eine cDNA zurückgeschrieben wird.

12. Verfahren nach irgendeinem der vorherigen Ansprüche, wobei der feste Träger aus der Gruppe ausgewählt ist, die aus Glas, einer elektronischen Vorrichtung, einem Siliziumträger, einem Kunststoffträger, einer CD (Compact Disc), einem Filter, einem Metallträger, mit Polylysinbeschichteten Flächen oder einer Mischung davon besteht.

13. Verfahren nach irgendeinem der vorherigen Ansprüche, wobei der erste Satz von (gegebenenfalls markierten) Nukleotidsequenzen (1) spezifisch an die Nukleotidzielsequenzen (3) in einer ersten Kammer (5) bindet,
- wobei die (gegebenenfalls markierten) Sequenzen des ersten Satzes von Nukleotidsequenzen (1), die an die Nukleotidzielsequenzen (3) gebunden sind, von der ersten Kammer (5) entfernt und in die zweite Kammer (6) überführt werden, und wobei die gegebenenfalls markierten Nukleotidsequenzen (1) des ersten Satzes von Nukleotidsequenzen durch Hybridisierung mit dem zweiten Satz von Capture-Nukleotidsequenzen (2) in der zweiten Kammer (6) nachgewiesen und/oder quantifiziert werden.

14. Diagnostischer Apparat und/oder Quantifizierungsapparat einer oder mehrerer homologer Nukleotidzielsequenzen, die möglicherweise in einer Probe vorhanden sind, der zwei getrennte Kammern (5 und 6), die auf einem festen Träger (4) vorhanden sind, und eine flüssige Verbindung zwischen den beiden Kammern (5,6) umfasst und wobei die zweite Kammer (6) Capture-Nukleotidsequenzen (2) enthält, die auf der Oberfläche des festen Trägers gemäß einer Anordnung von mindestens 4 diskreten Bereichen/cm² immobilisiert sind, wobei an jeden der diskreten Bereiche eine Art von Capture-Nukleotidsequenzen (2) gebunden ist.

15. Diagnostischer Apparat und/oder Quantifizierungsapparat nach Anspruch 14, der des Weiteren einen ersten Satz von (gegebenenfalls markierten) Nukleotidsequenzen (1) umfasst, die mit den in der ersten Kammer (5) nachzuweisenden und/oder zu quantifizierenden Nukleotidzielsequenzen reagieren können und die die in der zweiten Kammer (6) vorhandenen capture-Nukleotidsequenzen (2) hybridisieren können.

16. Apparat nach Anspruch 14 oder 15, wobei die beiden Kammern (5 und 6) auf einer einzigen Oberfläche eines festen Trägers (4) vorhanden sind.

17. Apparat nach irgendeinem der vorherigen Ansprüche 14 bis 16, wobei die beiden Kammern (5 und 6) durch eine kleine Röhre oder einen kleinen Kanal für die Lösungsübertragung von einer Kammer (5) in die andere Kammer (6) verbunden sind.

18. Maschine oder Automat für die Durchführung der verschiedenen Schritte des Verfahrens nach irgendeinem der vorherigen Ansprüche 1 bis 13 und umfassend den Apparat nach den Ansprüchen 14 bis 17.

## Revendications

1. Procédé pour une identification et/ou une quantification d'une ou plusieurs séquences nucléotidiques cibles (3) étant éventuellement présentes dans un échantillon et pour une discrimination à partir d'autres séquences homologues, en utilisant deux jeux de séquences nucléotidiques (1 et 2) ;
- dans lequel dans une première étape, un premier jeu de séquences nucléotidiques (1) (éventuellement marquées) comprises entre 8 et 60 bases, se lie spécifiquement auxdites séquences nucléotidiques cibles (3),
- dans lequel les séquences (éventuellement marquées) du premier jeu de séquences nucléotidiques (1) qui ne sont pas liées aux séquences nucléotidiques cibles (3) sont retirées,
- dans lequel les séquences nucléotidiques (éventuellement marquées) (1) du premier jeu de séquences nucléotidiques liées à la séquence nucléotidique cible (3) sont déshybridées les unes des autres et sont détectées et/ou quantifiées dans une deuxième étape par hybridation avec un second jeu de séquences nucléotidiques de capture (2) ayant au moins une partie de leur séquence nucléotidique complémentaire à la partie de la séquence nucléotidique des séquences (éventuellement marquées) (1) du premier jeu de séquences nucléotidiques qui se lie spécifiquement aux séquences nucléotidiques cibles (3), lesdites séquences nucléotidiques de capture (2) étant immobilisées sur la surface (4) d'un support solide selon un réseau d'au moins 4 régions discrètes/cm², chacune desdites régions discrètes étant liées avec une espèce des séquences nucléotidiques de capture (2), et
- dans lequel l'identification et la quantification de la liaison entre lesdites séquences nucléotidiques (éventuellement marquées) (1) et leurs séquences nucléotidiques de capture correspondantes (2) est corrélée avec l'identification et la quantification desdites séquences nucléotidiques cibles (3) présentes dans l'échantillon.

2. Procédé selon la revendication 1, dans lequel les séquences nucléotidiques cibles (3) sont d'abord liées sur un support solide (4), de préférence par une liaison covalente, avant leur liaison d'hybridation avec une ou plusieurs séquences (éventuellement marquées) (1) du premier jeu de séquences nucléotidiques.

3. Procédé selon la revendication 1 ou 2, dans lequel la liaison entre les séquences nucléotidiques cibles (3) et les séquences (éventuellement marquées) (1) du premier jeu de séquences nucléotidiques survient en solution.

4. Procédé selon l'une quelconque des revendications précédentes 1 à 3, dans lequel les séquences (éventuellement marquées) (1) du premier jeu de séquences nucléotidiques ont une longueur qui est essentiellement identique ou similaire à la longueur des séquences nucléotidiques de capture (2) du second jeu de séquences nucléotidiques.

5. Procédé selon l'une quelconque des revendications précédentes, dans lequel les séquences nucléotidiques cibles (3) à identifier et/ou quantifier présentent une homologie avec d'autres séquences homologues présentes dans l'échantillon, qui est supérieure à 30%, de préférence supérieure à 60%, de toute préférence supérieure à 80%.

6. Procédé selon l'une quelconque des revendications précédentes, dans lequel les séquences nucléotidiques cibles (3) à identifier et/ou quantifier différent des séquences homologues seulement par une base.

7. Procédé selon la revendication 1, dans lequel les séquences nucléotidiques de capture (2) du second jeu de séquences nucléotidiques ont une séquence qui est complémentaire aux séquences nucléotidiques (éventuellement marquées) (1) du premier jeu de séquences nucléotidiques sur leur séquence complète ou ont une séquence qui est liée au support solide par un espaceur ayant une longueur comprise entre 10 et 200 bases.

8. Procédé selon l'une quelconque des revendications précédentes, dans lequel les séquences nucléotidiques (éventuellement marquées) (1) contiennent un mélange de deux ou plusieurs séquences marquées terminées par un nucléotide différent qui se lie à la séquence nucléotidique cible adjacente à un éventuel SNP, un des nucléotides terminaux étant parfaitement assorti au SNP, et une autre sonde annexée, qui va se lier à l'autre site de la séquence cible SNP, la sonde parfaitement assortie étant ligaturée à cette séquence par une ligase avant d'être détectée sur une séquence nucléotidique de capture correspondante (2).

9. Procédé selon l'une quelconque des revendications précédentes, dans lequel les séquences nucléotidiques marquées (1) contiennent un mélange de sondes, chacune différant dans au moins une base située sur un site SNP et traitée avec une nucléase spécifique avant d'être détectée sur une séquence nucléotidique de capture correspondante.

10. Procédé selon l'une quelconque des revendications précédentes, dans lequel une ou plusieurs séquences nucléotidiques cibles (3) à détecter et/ou quantifier sont des ARNr, de préférence choisis dans le groupe constitué de ARNr 16S, 23S, 18S et 25S.

11. Procédé selon l'une quelconque des revendications précédentes 1 à 9, dans lequel une ou plusieurs séquences nucléotidiques cibles (3) à détecter et/ou quantifier sont des ARNm, de préférence un ARNm rétrotranscrit dans un ADNc par une séquence consensus.

12. Procédé selon l'une quelconque des revendications précédentes, dans lequel le support solide est choisi dans le groupe constitué de verre, d'appareil électronique, de support en silicium, de support plastique, de disque compact, de filtre, de support métallique, de surfaces couvertes de polylysine ou d'un mélange de ceux-ci.

13. Procédé selon l'une quelconque des revendications précédentes, dans lequel le premier jeu de séquences nucléotidiques (éventuellement marquées) (1) se lie spécifiquement aux séquences nucléotidiques cibles (3) dans une première chambre (5),
- où les séquences (éventuellement marquées) du premier jeu de séquences nucléotidiques (1) qui sont liées aux séquences nucléotidiques cibles (3) sont retirées de la première chambre (5) et transférées dans la seconde chambre (6) et où les séquences nucléotidiques éventuellement marquées (1) du premier jeu de séquences nucléotidiques sont détectées et/ou quantifiées par hybridation avec le second jeu de séquences nucléotidiques de capture (2) dans la seconde chambre (6).

14. Appareil de diagnostic et/ou de quantification d'une ou plusieurs séquences nucléotidiques cibles homologues, éventuellement présentes dans un échantillon, qui comprend deux chambres séparées (5 et 6) présentes sur un support solide (4), et une connexion liquide entre les deux chambres (5, 6) et dans lequel la seconde chambre (6) contient des séquences nucléotidiques de capture (2) immobilisées à la surface du support solide selon un réseau d'au moins 4 régions discrètes/cm², chacune desdites régions discrètes étant liées avec une espèce de séquences nucléotidiques de capture (2).

15. Appareil de diagnostic et/ou de quantification selon la revendication 14, qui comprend en outre un premier jeu de séquences nucléotidiques (éventuellement marquées) (1) capables de réagir avec les séquences nucléotidiques cibles à détecter et/ou quantifier dans la première chambre (5) et étant capables d'hybrider les séquences nucléotidiques de capture (2) présentes dans la seconde chambre (6).

16. Appareil selon la revendication 14 ou 15, dans lequel les deux chambres (5 et 6) sont présentes sur une seule surface du support solide (4).

17. Appareil selon l'une quelconque des revendications 14 à 16, dans lequel les deux chambres (5 et 6) sont reliées par un petit conduit ou canal pour le transfert de la solution d'une chambre (5) à l'autre chambre (6).

18. Machine ou automate pour exécuter les diverses étapes du procédé selon l'une quelconque des revendications précédentes 1 à 13 et comprenant l'appareil selon les revendications 14 à 17.
